# EUROPEAN PATENT APPLICATION

(11) **EP 1 130 021 A2**
(43) Date of publication of application: **05.09.2001**
(21) Application number: 01104474.0
(22) Date of filing: 28.02.2001
(51) Int. Cl.: C07D 491/16, C07D 471/04, C07D 491/22, C07K 16/44, G01N 33/68

(54) **Pyrrolopyridinium derivatives**

(30) Priority: 29.02.2000 JP 2000054245
(71) Applicant: NIPPON ZOKI PHARMACEUTICAL CO., LTD., Chuo-ku, Osaka (JP)
(72) Inventor: Nakamura, Ko, Nippon Zoki Pharmaceutical Co., Ltd., Yashiro-cho, Katoh-gun, Hyogo (JP); Horiuchi, Seikoh, Kumamoto-shi, Kumamoto (JP); Araki, Norie, Aso-gun, Komamoto (JP)
(74) Representative: HOFFMANN - EITLE

(57) **Abstract**

The present invention relates to a novel pyrrolopyridinium derivative of the general formulae (I), (II), and (III) 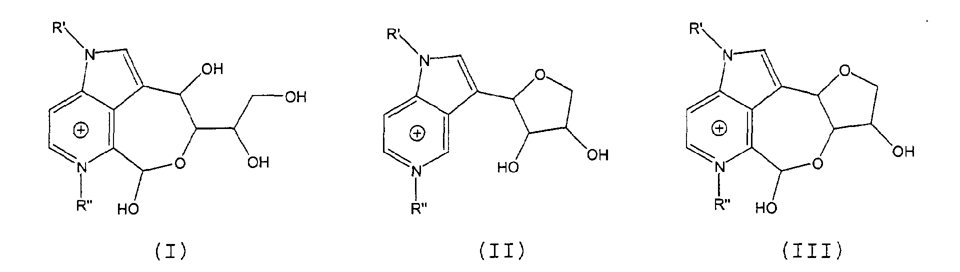 wherein R' and R'' independently represent a linear or branched alkyl group which may be substituted with an optionally protected amino group or a carboxyl group; including all stereoisomers and enantiomers, and salts thereof. Further, the invention concerns an antibody against this derivative as a hapten; a method for measuring this derivative using the antibody; and a test kit for the assay of said derivative compound comprising the antibody.

This derivative, antibody, method and test kit is useful for the in-vitro diagnosis of various diseases as well as for the evaluation of the effectiveness of pharmaceuticals against these diseases, e.g. diabetes, diabetic complications, renal failure, dialysis-related complications, aging, diseases accompanied by aging, Alzheimerdisease, etc. ; andamethod for diagnosis of diabetes, diabetic complications, renal failure, dialysis-related complications, aging, diseases accompanied by aging, Alzheimer disease, etc.

## Description

### [Technical Field]

The present invention relates to a novel pyrrolopyridinium derivative; an antibody against this derivative as a hapten; a method for measuring this derivative in a sample using the antibody; and a test kit for the assay of said derivative compound comprising the antibody.

This derivative, antibody, method and test kit is useful for the diagnosis of various diseases as well as for the evaluation of the effectiveness of pharmaceuticals against these diseases, e.g. diabetes, diabetic complications, renal failure, dialysis-related complications, aging, diseases accompanied by aging, Alzheimer disease, etc.; and a method for diagnosis of diabetes, diabetic complications, renal failure, dialysis-related complications, aging, diseases accompanied by aging, Alzheimer disease, etc.

### [Prior Art]

The known Maillard reaction may be classified into early stages wherein a Schiff base is formed from amino group in protein and aldehyde group in reduced sugar and is stabilized as a result of Amadori rearrangement, and an advanced stage wherein the above is transferred, after a long series of reactions, to Advanced Glycation Endproduct (AGE) which shows characteristic fluorescence, color change to brown and molecular crosslinking.

This reaction had been studied mainly in the field of food chemicals. However, in 1968, glycosylhemoglobin (HbAlc) which is one of the minor components of hemoglobin was identified in-vivo, and was found to be accumulated in patients suffering of diabetes, i.e. it was shown that the Maillard reaction could occur on a protein in the living body. With this as a momentum, the biological and medical meaning of Maillard reaction has been receiving public attentions.

In recent years, the relation between the AGE which is produced particularly in the advanced stage and various diseases has received public attention and the studies on AGE are in progress. Until now, several AGEs such as N^{ε}-carboxymethyllysine (CML), pentosidine, pyralin, N^{ε}-carboxyethyllysine (CEL), glyoxallysine dimer (GOLD) and methylglyoxallysine dimer (MOLD) have been reported. The present inventors and their co-workers have also found a pyridinium derivative called Crossline™ (Japanese Patent Laid-Open Hei-06/73057), naphthylidinium derivatives disclosed in Japanese Patent Laid-Open Hei-08/48686, pyrrolonaphthylidium derivatives disclosed in Japanese Patent Laid-Open Hei-10/158265, etc. as novel AGEs.

With regard to antibodies recognizing such an AGE, 6D12 (Kumamoto Antibody Laboratory) which is an anti-CML monoclonal antibody has been put on the market. Immmunohistological and immunochemical studies such as measurement of AGE concentrations, and immunological staining in human plasma, human serum, human urine, arteriosclerotic tissue, renal tissue of patient suffering from diabetic nephropathy, tissue of eye of aged people, cerebral tissue of patient suffering fromAlzheimer disease, etc. using antibodies of the above-mentioned AGEs have been actually carried out and studies for its significance of being have been further conducted.

With regard to the relation between AGE and various diseases, fluorescence, which is characteristic for AGE, was firstly used as an indicator for investigating the correlation between AGE and various diseases. In addition, studies have been performed using an antibody to the above-mentioned AGE whereby the participation of AGE in various diseases has been clarified. For example, refer to the review of S. Horiuchi (one of the present inventors), "AGE (glycation) and Diseases", BIO Clinica, 11(5), 314 (1996), and others.

For example, aging is thought to be a process where various proteins and DNA in tissues are modified or changed, such that they cannot be decomposed and removed, but are accumulated in the body, and finally the organism suffers a functional disorder. The advanced glycation of tissue proteins is exemplified as one of the causes for this phenomenon.

In fact, it has been shown that fluorescence of hydrolyzed products of skin collagen and eye lens proteins increases together with aging, and that an excessive advanced glycation of eye lens protein participates in the onset of cataract. In addition, AGE is now considered to be one of the essential causes for aging phenomena, since the inhibition of enzymatic activity and decreasing ability to decompose intracellular protein due to advanced glycation has been observed, as well as substance transport disorders caused by advancedly glycated cytoskeleton proteins, DNA mutations caused by AGE-induced cross-linking of DNA with proteins, etc. Also, it has been reported that anti-AGE antibodies react in a concentration-dependent manner with the water-soluble fraction and alkali-soluble fraction of the normal human lens crystalline, and a very high correlation was observed between the reactivity with the age. Further, an increase of advanced glycation in Descemet membrane and lens vesicle of cattle with aging was observed by immune electric microscopy, and a correlation of AGE immunoreaction of petrosal nerve cell with the age was shown by an immunohistochemical investigation using antibodies, etc.

It is known that, in diabetic patients, fluorescence is significantly increased as compared with healthy people. Moreover, accumulation of AGE was reported in renal tissues such as renal mesangium region, blood vessel wall and urinary tubule and coronary arteriosclerotic nest of diabetic patients. It was also reported that, as a result of immune staining using an anti-AGE antibody, AGE shows almost the same distribution in peripheral nerves of both experimental diabetic rats and human diabetes. Based on these findings, a correlation between diabetes mellitus and AGE has been recognized.

It has been also clarified that an excessive formation of AGE in organism is a cause of diabetic complications. For example, an advanced glycation of crystalline, which is an eye lens protein, is one of the causes of diabetic cataract. It was also shown that AGE is strongly related to diabetic nephropathy. It has been clarified that glomerular basement membrane collagen of kidney is advancedly glycated by hyperglycemia and that is apt to be bonded to albumin, immunoglobulin, low-density lipoprotein, etc. whereby the basement membrane thickens and renal filtration function is disturbed.

In addition, the possibility was pointed out AGE may be accumulated in mesangium cells, and that it participates in inflammation such as secretion of cytokine mediated by a receptor. Recently, it has been reported that a suppressor for the production of AGE is useful to diabetic nephropathy. It has been also reported that myelinprotein of nerve is advancedly glycated and, accordingly, it was suggested that AGE participates in diabetic neuropathy as well.

As such, the correlation of AGE with the onset of diabetic complications such as diabetic nephropathy, arteriosclerosis, neurosis, retinopathy, cataract, etc. has been clarified. AGE and diabetic complications are summarized, for example, in the review of Morisaki, et al., "AGE and Diabetic Complications", Saishin Igaku, 49 (2), 248 (1994).

In addition, the relation between AGE and dialysis-related amyloidosis has been recognized, based on the fact that fluorescence increases in the protein in serum of dialyzed patients, and that accumulation of AGE is noted at the site where amyloid of carpal tunnel of dialyzed patients is sedimented.

Further, it was reported that a scavenger receptor recognizing the AGE protein is present in monocytes, macrophages, mesangium cells and endothelial cells, and the AGE recognition mediated by such a receptor results in release of cytokine, promotion of blood vessel permeability, abnormal blood flow, etc., thus suggesting a relation of AGE with the symptoms of inflammation, capillary obstruction, arteriosclerosis, etc. Furthermore, it has been considered that AGE seriously participates in the onset and progress of Alzheimer disease because AGE is strongly detected in senile plaque, amyloid and neurofibril change which are characteristically noted in brains of patients suffering from Alzheimer disease.

Moreover, in recent years, free radicals (active oxygen) are receiving public attention as a cause of aging, tissue disorders of diabetes mellitus and generation of pathological changes of Alzheimer disease. It became clear that the Amadori compound produced by the initial-stage reaction of Maillard reaction is oxidized by active oxygen to afford AGE while it is a cause for generation of active oxygen as well. It was also reported in neuromatous cell strain (SK-N-SH) that neurofibril (PHF) tau-protein generates active oxygen by glycation, and that it activates the transcription factor NF-KB. It was further suggested that an extinction enzyme for active oxygen such as Cu-Zn-superoxide dismutase (Cu-Zn-SOD) is advancedly glycated, thus lowering its activity, which then becomes an essential cause for an increase in oxidative stress. Based thereon, the relation between Alzheimer disease and AGE has been suggested as well.

However, from the production processes for AGE as mentioned above, it is presumed that, in organisms, various kinds and types of AGE are produced depending on the circumstances, and that the distribution of such an AGE in tissues is different. It was the skilled person's desire to obtain many AGEs having various different properties for finding an optimum AGE suitable for each of such various kinds and types of circumstances in an organism. Accordingly, there was a demand for a novel AGE produced under a situation that is different from the conventional one, or, in other words, for a novel AGE having a structural skeleton different from the conventional AGE.

### [Matters to be solved by the Invention]

An object of the present invention is the provision of a novel pyrrolopyridinium derivative; an antibody against this derivative as a hapten; a method for measuring the presence of this derivative in a sample using the antibody; and a test kit for the assay of said derivative compound comprising the antibody. Especially, the method is a method for the in-vitro diagnosis of diabetes, diabetic complications, dialysis-related complications, aging, diseases accompanied by aging, or a method for the evaluation of the effectiveness of pharmaceuticals against diabetes-related diseases, aging, diseases accompanied by aging, etc.

The present inventors have conducted continued studies on the crosslinked substances, as an indicator to diagnose diabetic complications and aging, which are produced by both reactions of nonenzymatic glycation and oxidation of proteins. As a result, they found novel pyrrolopyridinium derivatives which have a completely new structural skeleton as compared with heretofore known AGEs whereby the present invention has been accomplished.

### [Detailed description of the Invention]

The novel pyrrolopyridinium derivatives (here and in the following of the present invention, if not otherwise indicated) are represented by the following formulae (I), (II) or (III); 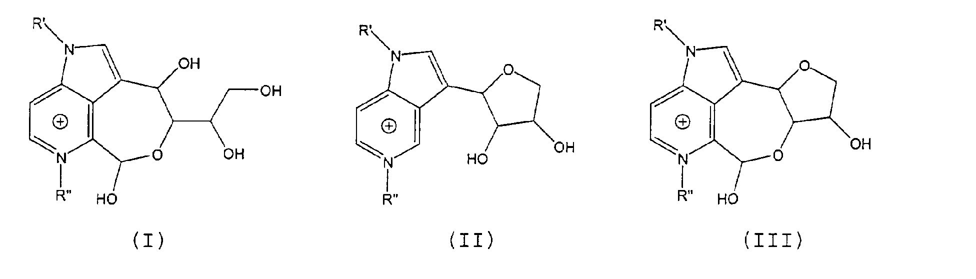 wherein R' and R'' independently represent a linear or branched alkyl group which may be substituted with an optionally protected amino group or a carboxyl group; including all stereoisomers and enantiomers, and salts thereof.

Among the various stereoisomers and enantiomers the compounds of the general formulae (I), (II) and (III) having the following stereoisomeric structure are especially preferred in the present invention. The formulae also show the numbering of the carbon atoms present therein as used in the present specification.

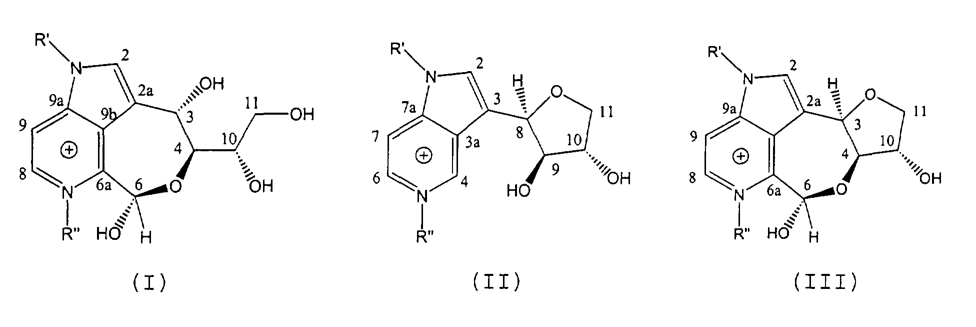

Preferred examples of the alkyl groups for R' and R'' in the above general formulae are linear or branched C₁₋₆-alkyl groups such as methyl, ethyl, propyl, isopropyl, butyl, isobutyl, sec-butyl, tert-butyl, pentyl, isopentyl, neopentyl, tert-pentyl, hexyl and dimethylbutyl.

The alkyl group may be substituted with amino, protected amino and/or carboxyl. Examples for the protective groups for the amino group are those which are commonly known and used in the field of peptide synthesis. Preferred examples are acetyl, benzyloxycarbonyl, p-methoxybenzyloxycarbonyl, p-chlorobenzyloxycarbonyl, p-nitrobenzyloxycarbonyl, p-phenylazobenzyloxycarbonyl, p-methoxyphenylazobenzyloxycarbonyl, tert-butoxycarbonyl (Boc), p-toluenesulfonyl (Tos), tert-amyloxycarbonyl, p-biphenylisopropyloxycarbonyl and diisopropylmethyloxycarbonyl and formyl.

Among the pyrrolopyridinium derivatives of the general formulae (I), (II) and (III), those wherein R' and R" are alkyl having amino and/or carboxyl can be easily combined with a carrier protein, and they are particularly useful for the preparation of the antibodies of the present invention. Regarding the hapten for preparation of antibody of the present invention, carriers for combining with the compounds (I), (II) or (III) are those the commonly-used in the field of art, such as proteins (e.g. serum albumin, keyhole lympet hemocyanin) and polymers (e.g. polylysine).

The pyrrolopyridinium derivatives include salts of the substances of the general formulae (I), (II) and (III). They are, for example, salts with inorganic acids such as hydrochloric acid, sulfuric acid, nitric acid, hydrobromic acid, phosphoric acid, perhydrochloric acid, thiocyanic acid, and boric acid, or with organic acids such as formic acid, acetic acid, haloacetic acid, propionic acid, glycolic acid, citric acid, tartaric acid, succinic acid, gluconic acid, lactic acid, malonic acid, fumaric acid, anthranilic acid, benzoic acid, cinnamic acid, p-toluenesulfonic acid, naphtalenesulfonic acid and sulfanilic acid.

Further examples are addition salts with ammonia or with organic bases such as organic amines; and salts with alkali metal such as sodium and potassium; alkali earth metal such as magnesium, calcium and barium; and other metals such as aluminum and zinc.

Those salts may be manufactured by known methods from the pyrrolopyridinium derivatives in a free state or may be mutually converted among the salts.

When there are stereoisomers such as cis-trans isomer, optical isomer, conformational isomer, hydrates and complexes for the substances of the present invention, the present invention includes any and all of them.

Aa an example of manufacturing methods for the substances of the present invention, a substance of the general formulae wherein R' is NH₂ or R' ' is NH₂ (amine component; wherein R' and R'' are the same groups as mentioned already) is reacted with a sugar, for example, hexose such as glucose, fructose, galactose, mannose or deoxyglucose, amino sugar such as glucosamine or galactosamine, oligosaccharide such as saccharose, to give the substance of the present invention. The substance of the present invention can also be manufactured by hydrolysis of the substance obtained by mixing together and reacting a substance of the general formulae (I), (II), or (III) with a protein or peptide as amine component.

The reaction conditions such as reaction temperature, reactiontime, pH, etc. are not particularly restricted, and may be established appropriately depending on the reacting compounds used. An easy procedure is to allow the reaction mixture to stand at room temperature. However, the reaction can be accelerated by heating and the like. The substances of the present invention prepared as such can be purified by common means such as distillation, chromatography, recrystallization, etc.

When the pyrrolopyridinium derivative is polymerized with bovine serum albumin (BSA) or with limpet blood dye protein (KLH) according to a method mentioned in e.g. Clin. Chem., 40(9), 1766-1773 (1994), etc. on an antibody to pentosidine, which is one of AGE, and then subjected to a common method such as to immunize to mouse, rabbit, or the like, a polyclonal or monoclonal antibody of the pyrrolopyridinium derivative can be prepared.

By preparing such an antibody and carrying out a known detection method such as ELISA or immunological staining, the present pyrrolopyridinium derivative can be measured in a sample. However, it can also be measured directly by HPLC or the like, e.g. by utilizing its fluorescent wave length.

The measured amount of the pyrrolopyridinium derivative in a sample as such is used as an index, and the degree of accumulation of the Advanced Glycation Endproducts in-vivo is evaluated, and can be taken as a basis for an evaluation of the degree of progress of diabetes mellitus, diabetic complications, dialysis-related complications, amyloidosis, aging or diseases accompanied by aging. It is also possible that, when the produced and accumulated amount of the pyrrolopyridinium derivative are measured in the presence and absence of a test drug against diabetes mellitus, diabetic complications, dialysis-related complications, amyloidosis, aging or diseases accompanied by aging, the pharmaceutical effect of this test drug can be evaluated.

With regard to the samples to be measured, living tissues, living tissue extracts, body fluid and/or urine may be exemplified; preferably, human skin tissues, human skin tissue extracts, human lens extracts, human arteriosclerotic tissues, renal tissues of patients suffering from diabetic nephropathy, tissues of aged people, cerebral tissues of patients suffering from Alzheimer disease, human blood, human plasma, human serum and human urine may be exemplified. Particularly preferred are human skin tissues, human skin tissue extracts, human plasma, human serum and human urine.

The embodiments and preferred embodiments of the present invention can be summarized as follows:
(1) A pyrrolopyridinium derivative of the above-mentioned formulae (I), (II) or (III) and salts thereof as defined in attended claims 1 to 3.
(2) An antibody as defined in attended claims 4 and 5.
(3) A method for the determination of a pyrrolopyridinium derivative mentioned under (1) as defined in attended claims 6 to 11.
(4) A test kit for the assay of a pyrrolopyridinium derivative mentioned under (1) as defined in attended claims 12 to 14.

### [Examples]

### Example 1

Glucose (79.2 g; 400 mM) and γ-aminobutyric acid (45.3 g; 400 mM) were dissolved in 1.1 L of a 250 mM phosphate buffer (pH 7.3) and allowed to stand at 37°C for 60 days.

The reaction solution (330 mL) was acidified to a pH of about 2 with trifluoroacetic acid (TFA) without concentrating, and then added to a column of Amberlite XAD-2 (Organo; 57 mm diameter × 750 mm) equilibrated with deionized water. This was further eluted with deionized water whereupon each 100 mL were fractionated. The resulting fractions were successively analyzed by HPLC and the fractions containing the compound 1 were collected, concentrated and evaporated to dryness . Then, it was added to Develosil ODS LOP-45S (Nomura Kagaku) equilibrated with 0.2% TFA and eluted with 7% acetonitrile/0.2% TFA. Each 15 mL of the eluates were collected by a fraction collector, each fraction was analyzed by reversed phase HPLC (column: Inertsil ODS-3V; 4.6mmdiameter×250mm; solvent: 20% methanol/0.2% TFA; flow rate: 0.8 mL/min), the fractions containing the compound 1 were collected, concentrated and evaporated to dryness, and the obtained fractions of raw product were repeatedly purified using Develosil ODS LOP-45S (5% acetonitrile/0.2% TFA).

Based on the analytical result by a reversed phase HPLC (column: Inertsil ODS-3V; 4.6 mm diameter × 250 mm; solvent: 20% methanol/0.2% TFA; flow rate: 0.8 mL/min), the fractions were divided into three groups depending on the components present therein, and two of them were further purified (Fr-1: 340 mg; Fr-2: 400 mg).

Fr-1 was subjected to reversed phase HPLC (column: Inertsil ODS-3; 20 mm diameter × 250 mm; flow rate: 10.0 mL/min; eluent: 8% acetonitrile/0.2%TFA) and, by means of detection of the UV absorption of 280 nm, thus obtaining a fraction containing the main component (8-(1,2-dihydroxyethyl)-6,9-dihydroxy-2,5-bis(3-carboxypropyl) -2,6,8,9-tetrahydro-7-oxa-2-aza-5-azoniabenzo[cd]azulene) (compound 1).

This was concentrated in vacuo and, when it became about 5 mL, it was added to Sep-Pak Vac C18 Cartridge equilibrated with deionized water, well washed with deionized water to remove trifluoroacetic acid and eluted with 50% acetonitrile. This was concentrated and evaporated to dryness to give the compound 1 (95 mg).

Fr-2 was subjected to a reversed HPLC (column: Inertsil OSD-3: 20 mm diameter × 250 mm; flow rate: 8.0 mL/min; eluent: 12% acetonitrile/0.2% TFA) and, by means of detection of the UV absorption of 280 nm, a fraction containing the main component was obtained. This was concentrated *in vacuo* and, when it became about 5 mL, the concentrate was added to Sep-Pak Vac C18 Catridge equilibrated with deionized water, well washed with deionized water to remove trifluoroacetic acid and eluted with 50% acetonitrile. This was concentrated and evaporated to dryness to give the compound 2 (3-(3,4-dihydroxytetrahydrofuran-2-yl)-1,5-bis(3-carboxypropyl )-1H-pyrrolo[3,2-c]pyridinium) (72 mg).

### Example 2

The compound 1 (19.6 mg) was dissolved in 3 mL of 2N HCl and stirred at room temperature. The reaction was checked from time to time by means of reversed phase HPLC (column: Inertsil ODS-3V: 4.6 mm diameter × 250 mm; flow rate: 0.8 mL/min; eluent: 12% acetonitrile/0.2% TFA) . After 12 hours, the reaction solution was concentrated in vacuo and evaporated to dryness, and the residue was purified by reversed phase HPLC (column: Inertsil ODS-3; 10 mm diameter × 250 mm; flow rate: 4.0 mL/min; eluent: 12% acetonitrile/0.2% TFA) . Each of the resulting material and product fractions was concentrated invacuo until about 5 mL, the concentrate was added to Sep-Pak Vac C18 Cartridge equilibrated with deionized water, well washed with deionized water to remove trifluoroacetic acid and eluted with 50% acetonitrile. The eluate was concentrated in vacuo and evaporated to dryness to give the compound 3 (8-(1,2-dihydroxyethyl)-1,5-bis(3-carboxypropyl)-2,6,8,9-tetra hydro-7-oxa-2-aza-5-azoniabenzo[cd]cyclopenta[h]azulene-6,8-diol) (6.9 mg).

### Example 3

Glucose (27.0 g; 200 mM) and N^{α}-acetyllysine (28.2 g; 200 mM) were dissolved in 750 mL of a 250 mM phosphate buffer (pH 7.3) and allowed to stand at 37°C for 56 days. Without concentration, the reaction solution was acidified to a pH of about 2 with trifluoroacetic acid and added to an ion-exchange resin of a sulfonate type (Diaion PK-216; Mitsubishi Chemical). The column was washed with water and eluted with 2N aqueous ammonia. The product fraction was concentrated and evaporated to dryness, added to Develosil ODS LOP-45S (Nomura Kagaku) equilibrated with 0.2% TFA and eluted with 10% methanol/0.2% TFA and 20% methanol/0.2% TFA where the methanol concentration was successively increased. The fraction which was eluted with 20% methanol/0.2% TFA was further purified by an STR ODS-II column (Shimadzu Techno Research) to give the compounds 4 and 5.

### Example 4

The same reaction of glucose as above was carried out using N^{α}-tert-butoxycarbonyl-L-lysine as an amine component, and the product was separated/purified, dissolved in TFA and allowed to stand for 30 minutes to remove a tertiary butoxycarbonyl group whereupon the compound 6 was prepared.

Physical properties of the resulting compounds of the present invention are shown below. Incidentally, labeled substances were manufactured using glucose where the carbon at the 1-position or all carbons was/were labeled with ¹³C by the same method as in the above Examples and used for the structural analysis. Fluorescent spectra and ultraviolet (UV) spectra were measured by a 650-4 Fluorescence Spectrophotometer (Hitachi) and a DU-650 (Beckman), respectively, inmethanol for both cases. Spattered ionmass analysis spectra (SIMS) were measured by an M-80B (Hitachi) using glycerol as a matrix. Nuclear magnetic resonance (NMR) spectra were measured inheavywaterusinganARX-500 (Bruker) using a¹ Hresonance frequency of 500.13 MHz and a ¹³C resonance frequency of 125.77 MHz as 0.00 ppm. Assignments of ¹H- and ¹³C-NMR spectra were determined by two-dimensional NMR such as ¹H-¹H COSY, HMQC and HMBC.

### Compound 1

Fluorescent spectrum: EXmax = 325 nm, EMmax = 440 nm SIMS: m/z 439
NMR spectrum: shown in Table 1

**Table 1**

| (MeOH-*d4)* | | | |
|---|---|---|---|
| C# | ¹³C (ppm) | ¹H (ppm) | Hz |
| 3'^{a} | 25.2 | * | m |
| 3"^{a} | 26.9 | * | m |
| 2'^{b} | 30.5 | * | m |
| 2"^{b} | 30.6 | * | m |
| 4' | 46.0 | 4.44 | t (7) |
| 4" | 55.4 | 4.55/4.82 | m,m |
| 11a | 63.4 | 3.70 | dd(7,12) |
| 11b | 63.4 | 3.95 | dd(3,12) |
| 3 | 65.0 | 5.39 | s |
| 10 | 70.1 | 3.98 | ddd(3,7,9) |
| 4 | 72.4 | 4.54 | d(9) |
| 6 | 91.5 | 6.80 | s |
| 9 | 108.0 | 8.07 | d(7) |
| 2a | 120.5 | | |
| 9b | 121.2 | | |
| 8 | 135.6 | 8.47 | d(7) |
| 2 | 135.8 | 7.97 | s |
| 9a | 142.0 | | |
| 6a | 149.8 | | |
| COOH'^{c} | 175.3 | | |
| COOH"^{c} | 175.5 | | |

| | | | |
|---|---|---|---|
| a-c : Assignments with the same letter C# may be interchanged | | | |
| * : not assigned | | | |

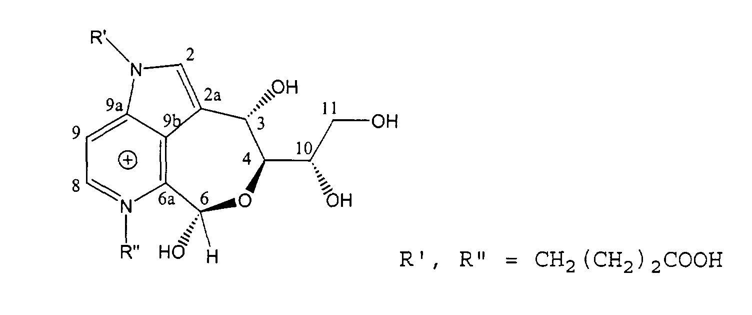

### Compound 2

Fluorescent spectrum: EXmax = 331 nm, EMmax = 466 nm SIMS: m/z 393
NMR spectrum: shown in Table 2

**Table 2**

| (MeOH-*d4)* | | | |
|---|---|---|---|
| C# | ¹³C (ppm) | ¹H (ppm) | Hz |
| 3'^{a} | 25.3 | * | m |
| 3"^{a} | 26.8 | * | m |
| 2'^{b} | 30.6 | * | m |
| 2"^{b} | 30.7 | * | m |
| 4' | 46.0 | 4.42 | t (7) |
| 4" | 59.5 | 4.65 | br.t(7) |
| 10 | 70.9 | 4.32 | ddd(2,5,5) |
| 11a | 73.6 | 3.93 | dd(2,10) |
| 11b | 73.6 | 4.42 | dd(5,10) |
| 8 | 76.3 | 5.01 | d(8) |
| 9 | 77.5 | 4.11 | dd(5,8) |
| 7 | 108.3 | 8.07 | d(7) |
| 3 | 119.1 | | |
| 3a | 123.5 | | |
| 2 | 132.3 | 7.85 | s |
| 6 | 133.9 | 8.45 | dd(1,7) |
| 4 | 138.6 | 9.24 | d(1) |
| 7a | 141.6 | | |
| COOH'^{c} | 175.3 | | |
| COOH"^{c} | 175.4 | | |

| | | | |
|---|---|---|---|
| a-c : Assignments with the same letter C# may be interchanged | | | |
| * : not assigned | | | |

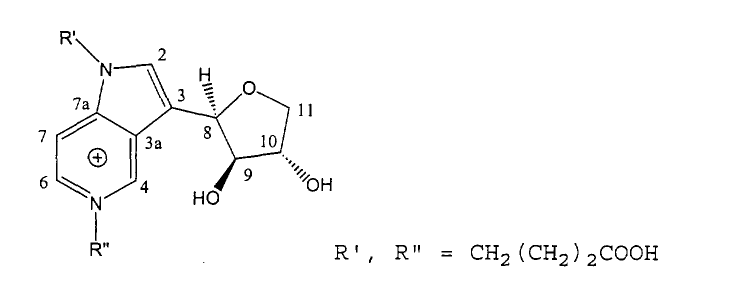

### Compound 3

Fluorescent spectrum: EXmax = 324 nm, EMmax = 453 nm SIMS: m/z 421 NMR spectrum: shown in Table 3

**Table 3**

| (MeOH-*d4)* | | | |
|---|---|---|---|
| C# | ¹³C (ppm) | ¹H (ppm) | Hz |
| 3'^{a} | 25.3 | * | m |
| 3"^{a} | 27.1 | * | m |
| 2'^{b} | 30.8 | * | m |
| 2"^{b} | 31.1 | * | m |
| 4' | 46.1 | 4.43 | t (7) |
| 4" | 55.2 | 4.59/4.80 | m,m |
| 4 | 70.6 | 5.20 | dd(2,5) |
| lla | 70.9 | 3.76 | dd(8,8) |
| 11b | 70.9 | 4.14 | dd(8,8) |
| 10 | 71.8 | 4.70 | ddd(5,8,8) |
| 3 | 75.9 | 5.26 | d(2) |
| 6 | 90.9 | 6.92 | s |
| 9 | 108.1 | 8.08 | d(7) |
| 2a | 115.1 | | |
| 9b | 121.3 | | |
| 8 | 135.6 | 8.47 | d(7) |
| 2 | 136.8 | 7.96 | s |
| 9a | 141.8 | | |
| 6a | 150.1 | | |
| COOH'^{c} | 175.6 | | |
| COOH"^{c} | 176.0 | | |

| | | | |
|---|---|---|---|
| a-c : Assignments with the same letter C# may be interchanged | | | |
| * : not assigned | | | |

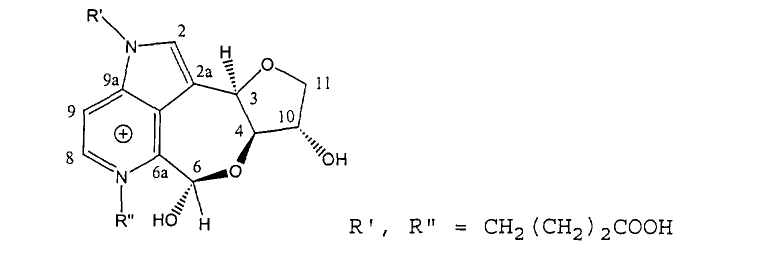

### Compound 4

Fluorescent spectrum: EXmax = 325 nm, EMmax = 440 nm SIMS: m/z 609 NMR spectrum: shown in Table 4

**Table 4**

| (MeOH-*d4*) | | | |
|---|---|---|---|
| C# | ¹³C (ppm) | ¹H (ppm) | Hz |
| 4' | 48.4 | 4.37 | t(6) |
| 4" | 57.6 | 4.0-4.1 | m |
| 11a | 64.6 | 3.71 | dd(7,12) |
| 11b | 64.3 | 3.97 | dd(3,12) |
| 3 | 66.6 | 5.46 | s |
| 10 | 71.6 | 3.98 | m |
| 4 | 74.0 | 4.55 | d(9) |
| 6 | 93.0 | 6.77 | s |
| 9 | 110.0 | 7.97 | d(7) |
| 2a | 120.2 | | |
| 9b | 122.7 | | |
| 8 | 137.6 | 8.35 | d(7) |
| 2 | 137.9 | 7.95 | s |
| 9a | 143.8 | | |
| 6a | 149.5 | | |

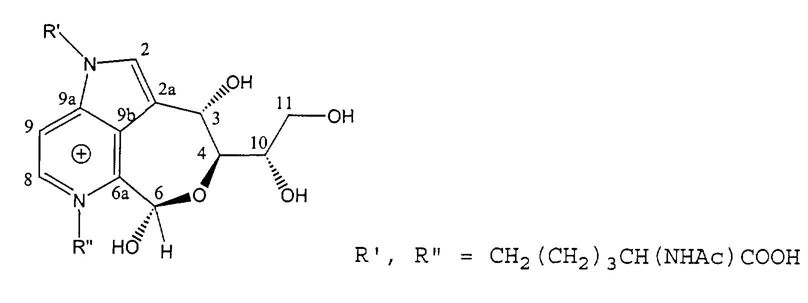

### Compound 5

Fluorescent spectrum: Exmax = 324 nm, Emmax = 453 nm SIMS: m/z 591
NMR spectrum: shown in Table 5

**Table 5**

| (MeOH-*d*4) | | | |
|---|---|---|---|
| C# | ¹³C (ppm) | ¹H (ppm) | Hz |
| 4' | 48.5 | 4.37 | t(6) |
| 4" | 57.1 | 4.63 | m |
| 11a | 72.1 | 3.75 | dd(8,8) |
| 11b | 72.1 | 4.17 | dd(8,13) |
| 3 | 77.8 | 5.35 | d(1) |
| 10 | 72.9 | 4.85 | ddd(5,8,13) |
| 4 | 72.4 | 5.22 | dd(1,5) |
| 6 | 92.6 | 6.89 | s |
| 9 | 110.2 | 7.98 | d(7) |
| 2a | 120.2 | | |
| 9b | 123.0 | | |
| 8 | 137.6 | 8.39 | d(7) |
| 2 | 139.3 | 7.96 | s |
| 9a | 143.7 | | |
| 6a | 149.7 | | |

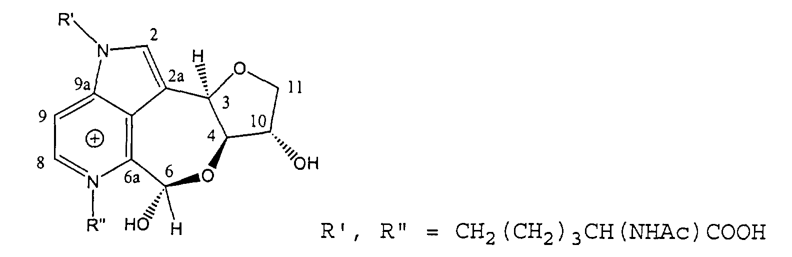

### Example 5

The antibody against the present pyrrolopyridinium derivative was prepared by a usual method, i.e., the substance combined with keyhole lympet hemocyanin was administered to immunize a rabbit. The resulting antibody significantly reacted with various glycated proteins prepared in vitro, while it did not react with normal non-glycated proteins. Additionally, it became clear that the antibody reacts with extracts of human eye lens and the reaction is increasing according to aging.

### [Merit of the Invention]

The pyrrolopyridinium derivative of the present invention is a novel AGE having entirely new structural skeleton different from the heretofore known AGE, and, based on its characteristic structure, it is believed to be a cross-linked substance produced by both glycation reaction and oxidation reaction. In addition, it has a hemiacetal structure as a reactive site in the molecule, and is much noteworthy as a new AGE.

As mentioned already, it has been clarified from the result of various studies that, as compared with healthy people, diabetic patients and dialyzed patients are under a high oxygen stress, and a cross-linked substance like the substance of the present invention, which is produced not only by a glycation reaction but also by an oxidation reaction, has a very high utility as a new index for onset/progress of diabetic complications, dialytic complications or aging.

Accordingly, it is now possible, using the substance of the present invention as an index, to conduct a test for diabetes mellitus, diabetic complications such as diabetic nephropathy, diabetic arteriosclerosis, diabetic neurosis, diabetic cataract, diabetic retinopathy, etc., dialysis-related complications, Alzheimer disease and aging and diseases accompanied with aging. Also, it is now possible to determine the effectiveness of a pharmaceutical against these diseases using the substance of the present invention as an index.

Further, an antibody against the substance of the present invention as a hapten can be immunochemically and immunohistochemically utilized in the above-mentioned test.

The present pyrrolopyridinium derivative is considered to be present in an organism and to have a different bioactivity as compared with conventional AGE, thusrendering it very useful in the present technical field where a great variety of AGE has been demanded.

## Claims

1. Pyrrolopyridinium derivative according to any of the general formulae (I), (II) or (III) 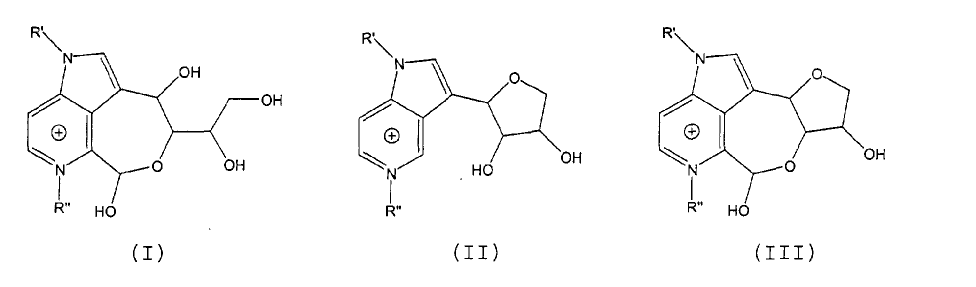 wherein R' and R" independently represent a linear or branched alkyl group which may be substituted with an optionally protected amino group or a carboxyl group; including all stereoisomers and enantiomers, and salts thereof.

2. Pyrrolopyridinium derivative of claim 1, wherein the compounds of the general formulae (I), (II) and (III) have the following stereoisomeric structure

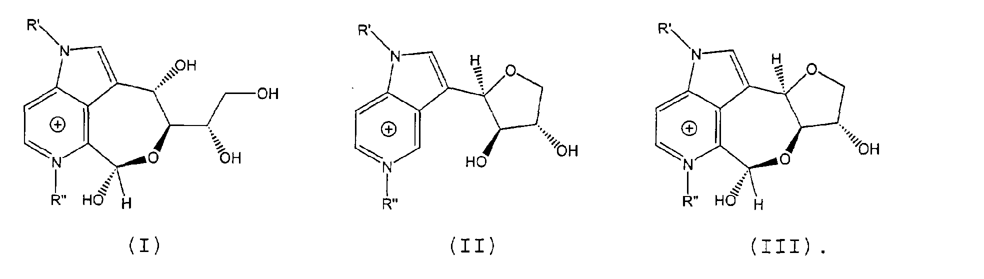

3. Pyrrolopyridinium derivative of claim 1 or 2, wherein R' and R' ' independently represent a linear or branched C₁₋₆-alkyl group which may be substituted with an optionally protected amino group or a carboxyl group.

4. Antibody against the compound of any of claims 1-3, optionally together with a suitable carrier, as hapten.

5. Antibody of claim 4, which is a monoclonal antibody or a polyclonal antibody.

6. Method for the determination of the presence of a compound of any of claims 1-3 in a sample using the antibody of any of claims 4-5.

7. Method of claim 6, wherein the reaction product with a compound as defined in any of claims 1-3 is measured by a fluorescence technique.

8. Method of any of claims 6-7, wherein the sample is selected from living tissue, an extract from living tissue, body fluid, blood, plasma, serum and urine.

9. Method of claim 8, wherein the sample is derived from a human.

10. Method of any of claims 6-9, which is a method for the in-vitro diagnosis of diseases and/or physical conditions connected with a change of the concentration of any of the compounds defined in any of claims 1-3 in the living body, or for the evaluation of the effectiveness of a medicament in the treatment of these diseases and/or physical conditions.

11. Method of claim 10, wherein the diseases and/or physical conditions are selected from diabetes, diabetic complications, dialysis related complications, amyloidosis, aging and diseases accompanied with aging.

12. Test kit for the assay of a compound of any of claims 1-3, comprising an antibody according to any of claims 4-5.

13. Test kit of claim 12, which is a test kit for the diagnosis of diseases and/or physical conditions connected with a change of the concentration of any of the compounds defined in any of claims 1-3 in the living body, or for the evaluation of the effectiveness of a medicament in the treatment of these diseases and/or physical conditions.

14. Test kit of claim 12 or 13, wherein the diseases and/or physical conditions are selected from diabetes, diabetic complications, dialysis related complications, amyloidosis, aging and diseases accompanied with aging.
